(19) Europäisches Patentamt
European Patent Office
Office européen des brevets

(11) Veröffentlichungsnummer : **0 459 196 B1**

(12) **EUROPÄISCHE PATENTSCHRIFT**

(45) Veröffentlichungstag der Patentschrift : **30.03.94 Patentblatt 94/13**

(51) Int. Cl.$^5$ : **C07C 255/51**, C07C 253/14

(21) Anmeldenummer : **91107662.8**

(22) Anmeldetag : **11.05.91**

# (54) Fluormethylierte Polycyanbenzole, deren Alkalicyanid-Addukte, Verfahren zu ihrer Herstellung und Verwendung der fluormethylierten Polycyanbenzole.

(30) Priorität : **26.05.90 DE 4016993**
**18.07.90 DE 4022751**

(43) Veröffentlichungstag der Anmeldung : **04.12.91 Patentblatt 91/49**

(45) Bekanntmachung des Hinweises auf die Patenterteilung : **30.03.94 Patentblatt 94/13**

(84) Benannte Vertragsstaaten : **CH DE FR GB LI**

(56) Entgegenhaltungen :
**DE-A- 3 718 641**
**US-A- 4 517 129**

(73) Patentinhaber : **BAYER AG**
**D-51368 Leverkusen (DE)**

(72) Erfinder : **Beck, Gunther, Dr.**
**Am Mittelberg 19**
**W-5090 Leverkusen 1 (DE)**
Erfinder : **von der Emden, Wolfgang, Dr.**
**Nittumer Weg 71**
**W-5060 Bergisch Gladbach 2 (DE)**
Erfinder : **Heitzer, Helmut, Dr.**
**Höhenstrasse 84**
**W-5090 Leverkusen 3 (DE)**
Erfinder : **Kröck, Friedrich Wilhelm, Dr.**
**Ackerstrasse 45**
**W-5068 Odenthal (DE)**
Erfinder : **Kysela, Ernst, Dr.**
**Virchow-Strasse 14**
**W-5060 Bergisch Gladbach 1 (DE)**
Erfinder : **Marhold, Albrecht, Dr.**
**Carl-Duisberg-Strasse 329**
**W-5090 Leverkusen 1 (DE)**

**EP 0 459 196 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue fluormethylierte Polycyanbenzole, ein Verfahren zur ihrer Herstellung, die bei diesem Herstellungsverfahren als Zwischenverbindungen anfallenden salzartigen Alkalicyanid-Addukte der fluormethylierten Polycyanbenzole und die Verwendung der fluormethylierten Polycyanbenzole für den Nachweis von Anionen.

Es wurden neue fluormethylierte Polycyanbenzole der Formel

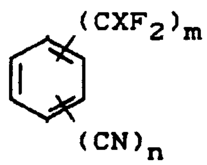

(I)

gefunden, in der

X für Wasserstoff, Fluor oder Chlor steht,

m 1 oder 2 ist und

n (6 -m) ist.

Die Erfindung betrifft ferner ein Verfahren zur Herstellung der fluormethylierten Polycyanbenzole der Formel (I), wonach man fluormethylierte Fluorbenzole der Formel

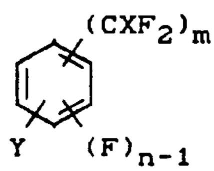

(II),

in der X, m und n die unter Formel (I) angegebene Bedeutung haben und

Y für Fluor oder CN steht,

mit Alkalicyaniden in organischen Verdünnungsmitteln zu salzartigen Addukten der Formel

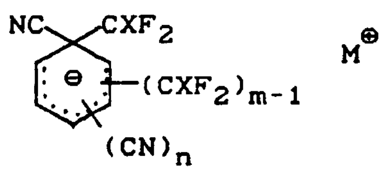

(III),

in der X, m und n die unter Formel (I) angegebene Bedeutung haben und $M^{\oplus}$ für ein Alkalimetall-Kation steht, umsetzt, und dieses Addukt, gegebenenfalls ohne es zu isolieren, durch Behandeln mit Säuren oder durch Erhitzen auf Temperaturen von 150-300°C, vorzugsweise im Vakuum, in die Verbindungen der Formel (I) überführt.

Die Erfindung betrifft außerdem die salzartigen Addukte der Formel (III).

In der ersten Reaktionstufe des erfindungsgemäßen Verfahrens werden als Alkalicyanide vorzugsweise die wohlfeilen Cyanide des Natriums oder Kaliums verwendet.

Die Umsetzung mit den Alkalicyaniden wird in organischen Verdünnungsmitteln vorgenommen. Als organische Verdünnungsmittel werden übliche aprotische polare bzw. dipolare Lösungsmittel eingesetzt. Beispiele für solche Lösungsmittel sind:

Aliphatische Nitrile wie Acetonitril, Propionitril, 3-Methoxy-propionitril; aliphatische und cyclische Ether wie Ethylenglykoldimethylether, Diethylenglykoldimethylether (Diglyme), Tetrahydrofuran; N,N-Dialkylamide niederer aliphatischer Carbonsäuren, wie N,N- Dimethylformamid, N,N-Diethylacetamid, N-Methylpyrrolidon; aliphatische Sulfoxide wie Dimethylsulfoxid; aliphatische Sulfone wie Dimethylsulfon und Tetramethylensulfon; ferner Tetramethylharnstoff, Ehylencarbonat, Propylencarbonat, N,N'-Dimethyl-1,3-imidazolin-2-on und Hexamethylphosphorsäuretrisamid. Besonders bevorzugt sind Acetonitril und N,N-Dimethylformamid.

Die Umsetzung der fluormethylierten Fluorbenzole der Formel (II) mit den Alkalicyaniden wird bei Temperaturen von -50°C bis +150°C, vorzugsweise von 0°C bis 100°C, besonders bevorzugt von 10°C bis 50°C

ausgeführt.

Die Reaktion wird im allgemeinen bei Normaldruck vorgenommen.

Die erfindungsgemäße Umsetzung der Verbindungen der Formel (II) mit den Alkalicyaniden läßt sich durch folgende Reaktionsgleichungen beschreiben:

(a) bei Verwendung von Difluoromethyl-pentafluorobenzol:

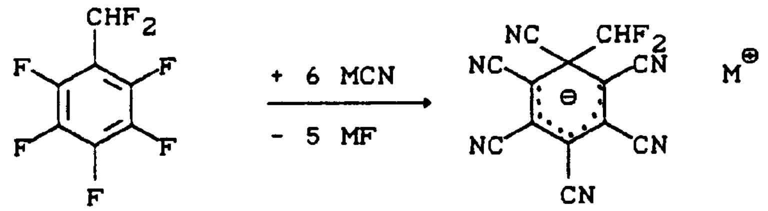


(a)

(b) bei Verwendung von α,α,α,2,3,5,6-Heptafluoro-p-tolunitril:

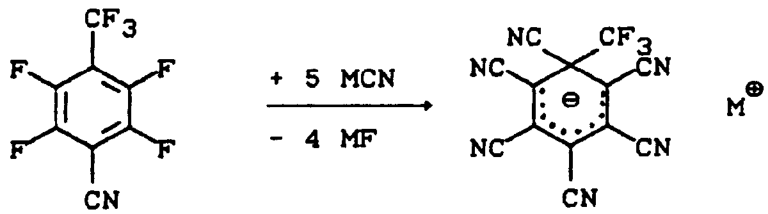


(b)

Wie aus den vorstehenden Reaktionsgleichungen (a) und (b) hervorgeht, sind zur Herstellung der Addukte der Formel (III) in Abhängigkeit von der Anzahl der an den Benzolring gebundenen und erfindungsgemäß gegen Cyangruppen auszutauschenden Fluoratome 5 oder 6 Mol Alkalicyanid je Mol Ausgangsverbindung (II) erforderlich. Statt der stöchiometrisch erforderlichen Menge Alkalicyanid kann man jedoch auch beliebige Unterschüsse an Alkalicyanid verwenden, da sich die nicht umgesetzten Ausgangsverbindungen (II) in ihren Löslichkeitseigenschaften wesentlich von den Addukten (III) unterscheiden und sich deshalb in einfacher Weise, z.B. durch Behandeln mit Lösungsmitteln wie Dichlormethan, von den in diesen Lösungsmitteln völlig unlöslichen Addukten der Formel (III) abtrennen lassen. Das gleiche gilt, wenn man die durch die Reaktionsgleichungen (a) und (b) beschriebenen Umsetzungen vor ihrer Beendigung abbricht.

Die Reaktionszeiten für die Adduktbildung schwanken je nach eingesetztem Fluorbenzol der Formel (II), den angewandten Reaktionstemperaturen und dem Lösungsmittel zwischen einer Stunde und zwei Wochen.

Die für das erfindungsgemäße Verfahren als Ausgangsverbindungen benötigten fluormethylierten Fluorbenzole der Formel (II) sind bekannt und z.B. in J. Fluorine Chem. 1987, 37, Seite 1 bis 14 beschrieben oder können nach an sich bekannten Verfahren hergestellt werden.

Die erfindungsgemäße Umsetzung der Alkalicyanide mit den Verbindungen der Formel (II) wird vorzugsweise so ausgeführt, daß man die Lösung des fluormethylierten Fluorbenzols der Formel (II) im betreffenden wasserfreien Lösungsmittel unter Feuchtigkeitsausschluß und unter Rühren bei Temperaturen von -40°C bis Raumtemperatur mit der stöchiometrisch erforderlichen Menge Alkalicyanid (oder einem Unterschuß) versetzt und bei der gewählten Endtemperatur von z.B. 20°C bis 80°C so lange weiterrührt, bis die (gaschromatographisch leicht verfolgbare) Reaktion vollständig oder weitgehend abgelaufen ist.

Wünscht man die Zwischenisolierung der Adukkte der Formel (III), so geht man bevorzugt folgendermaßen vor:

Nach beendeter Umsetzung der Verbindungen der Formel (II) mit den Alkalicyaniden filtriert man von etwa ungelöst gebliebenen Resten ab; das Filtrat wird im Vakuum unter milden Bedingungen (Raumtemperatur bis 50°C) vom Lösungsmittel befreit. Der Rückstand wird zum Entfernen gegebenenfalls nicht umgesetzter Reste der Ausgangsverbindungen (II) bei Raumtemperatur mit einem Lösungsmittel z.B. Dichlormethan, verrührt. Die nach dieser Behandlung mit Lösungsmitteln ungelöst zurückbleibenden orangeroten bis roten Alkalisalze der Formel (III) werden abfiltriert; aus dem Filtrat kann das Ausgangsmaterial der Formel (II) zurückgewonnen werden.

Die Alkalisalze der Formel (III) sind bei Raumtempertur stabile Verbindungen; sie liefern fluoreszierende Lösungen.

Die Addukte der Formel (III) lassen sich entweder durch Behandeln mit Säuren oder thermisch, durch Erhitzen auf Temperaturen von 150°C bis 300°C, vorzugsweise von 170°C bis 230°C, vorzugsweise im Vakuum, in die Verbindungen der Formel (I) überführen.

Für die Spaltung der Verbindungen der Formel (III) mit Säuren werden vorzugsweise flüssige, niedere aliphatische Carbonsäuren, wie Essigsäure, oder aber anorganische Mineralsäuren, wie Salzsäure, verwendet. Die saure Spaltung wird vorzugsweise bei Raumtemperatur vorgenommen. Zur Spaltung verrührt man die Verbindungen der Formel (III) mit den flüssigen Säuren, bis die charakteristische rote Farbe der zunächst entstehenden Lösungen verschwunden ist. Anschließend wird das Reaktionsgemisch mit Wasser verdünnt und der Niederschlag abfiltriert. Die Säurespaltung kann durch folgende Reaktionsgleichung beschrieben werden:

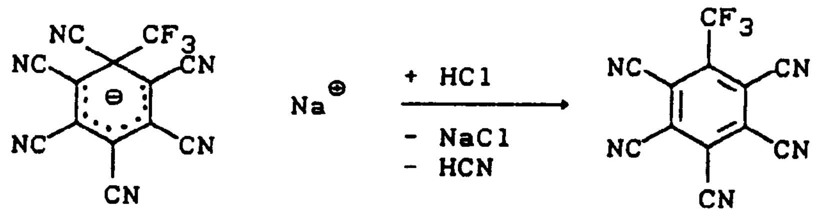


(c)

Aus der Reaktionsgleichung geht hervor, daß zur Spaltungsreaktion je Mol Alkalisalz der Formel (III) ein Äquivalent Säure erforderlich ist. Da man die Säure zugleich als Reaktionsmedium verwenden kann, werden die Säuren vorteilhaft im Überschuß angewandt. Im allgemeinen hat es sich bewährt, je Gew.-Teil Alkalisalz der Formel (III) 1 bis 50 Vol.-Teile Säure einzusetzen.

Bei der sauren Spaltung der isolierten Alkalisalze der Formel (III) ist Verwendung 1-normaler wäßriger Salzsäure besonders bevorzugt.

In Abhängigkeit vom angewandten Mengenverhältnis Alkalisalz der Formel (III)/Säure und der Konzentration der verwendeten Säure ist die Spaltung bei Raumtemperatur in 0,1 bis 24 Stunden beendet.

Zur thermischen Spaltung der Alkalisalze der Formel (III) werden diese auf Temperaturen von 150°C bis etwa 300°C, vorzugsweise 170°C bis 230°C erhitzt. Vorzugsweise führt man die thermische Spaltung im Vakuum (z.B. im Bereich von 10 bis 0,01 mbar) in einer Sublimationsapparatur durch. Man erhält auf diese Weise die erfindungsgemäßen fluormethylierten Polycyanbenzole der Formel (I) in reiner Form. Je nach Art des Alkalisalzes der Formel (I) und der gewählten Zersetzungstemperatur erfordert die thermische Spaltung 0,5 bis 24 Stunden.

Zur Herstellung der erfindungsgemäßen fluormethylierten Polycyanbenzole der Formel (I) ist die Isolierung der Addukte der Formel (III) nicht erforderlich. Man kann die Umsetzung der Ausgangsverbindungen der Formel (II) mit den Alkalicyaniden und die anschließende saure Spaltung der bei dieser Umsetzung entstandenen Alkalisalze der Formel (III) auch in einer Art "Eintopfreaktion" durchführen. Hierzu wird nach Beendigung der ersten Reaktionsstufe die Reaktionsmischung mit dem etwa 5 bis 10-fachen ihres Volumens an Wasser und gleichzeitig oder unmittelbar danach mit einer Mineralsäure, vorzugsweise Salzsäure, versetzt und 0,5 bis 24 Stunden gerührt. Anschließend werden die fluormethylierten Polycyanbenzole der Formel (I), die in diesen wäßrigen Medien schwer löslich sind, durch Filtration isoliert. Falls gewünscht oder erforderlich, können die so erhaltenen Verbindungen der Formel (I) üblichen Reinigungsoperationen, wie Vakuumsublimation (z.B. bei 200°C/0,1 mbar), Umkristallisation und dergleichen, unterworfen werden.

Der glatte Verlauf der Umsetzung der fluormethylierten Polyfluorbenzole der Formel (II) mit Alkalicyaniden zu den isolierbaren, stabilen Addukten der Formel (III) und deren Umsetzung zu den erfindungsgemäßen Verbindungen der Formel (I) ist außerordentlich überraschend, weil aus der Literatur bekannt ist, daß bei der Umsetzung von z.B. Hexafluorobenzol mit Natriumcyanid in Dimethylformamid keine identifizierbaren Produkte entstehen; alle Versuche zur Isolierung von Hexacyanobenzol waren erfolglos (s. J. Chem. Soc. (C), 1966, 708).

Polycyanbenzole sind wichtige Ausgangsmaterialien für die Herstellung von Charge-Transferkomplexen; diese CT-Komplexe zeichnen sich durch eine sehr gute elektrische Leitfähigkeit aus (s. z.B. Bull Acad. Polon. Sci. 23, 563 (1975)). Es besteht daher Interesse an einem wirtschaftlichen Herstellungsverfahren für diese Verbindungsklasse. Die hier erstmals synthetisierten fluormethylierten Polycyanbenzole der Formel (I) ergeben z.B. mit Pyren tief dunkel gefärbte Charge-Transfer-Komplexe mit interessanten Materialeigenschaften. Darüber hinaus zeigen die Polycyanbenzole der Formel (I) biozide, insbesondere insektizide und fungizide Wirkungen.

Die Alkalisalze der Formel (III) können in Polymere eingearbeitet werden, die dann mit orangeroter Farbe fluoreszieren.

Hierzu löst man z.B. Polyacrylnitril und ein Alkalisalz der Formel (III) in Dimethylformamid und verdampft

anschließend das Lösungsmittel.

In ähnlicher Weise löst man z.B. Polyvinylalkohol und ein Alkalisalz der Formel (III) in Wasser und verdampft anschließend das Lösungsmittel.

Weiterer Gegenstand der Erfindung ist die Verwendung der fluormethylierten Polycyanobenzole der Formel (I) zum Sichtbarmachen und zur qualitativen und/oder quantitativen Bestimmung von Anionen.

Obwohl für den Nachweis von Kationen geeignete Reagentien in größerer Zahl bekannt sind (sh. z.B. die als Nachweis-Reagentien für Schwermetallkationen verwendeten Dithizone), fehlte es bislang an universell verwendbaren, d.h. für den Nachweis von Anionen der verschiedensten Säuren geeigneten Reagentien.

Es wurde nun aber gefunden, daß die erfindungsgemäßen fluormethylierten Polycyanbenzole (I) mit Anionen Komplexanionen bilden, die das Licht im sichtbaren und/oder UV-Bereich stark absorbieren und die sich deshalb ausgezeichnet zum Sichtbarmachen und zur qualitativen und quantitativen Bestimmung von Anionen eignen.

Das Sichtbarmachen oder die Bestimmung der Anionen mit den erfindungsgemäß als Nachweis-Reagentien zu verwendenden fluormethylierten Polycyanbenzolen der Formel I geschieht in der Weise, daß man die Salze, die die zu bestimmende Anionen enthalten, oder die Lösungen dieser Salze mit Lösungen der fluormethylierten Polycyanbenzole der Formel I in Berührung bringt, z.B. durch Vermischen der Salzlösungen mit der Lösung der Polycyanbenzole der Formel I oder durch Besprühen der (auf feste Träger aufgebrachten) Salze mit den Lösungen der fluormethylierten Polycyanbenzole der Formel I.

Da sich Salze bei der chromatographischen Untersuchung voneinander durch ihren $R_f$-Wert unterscheiden und das Erkennen der Salze auf der stationären Phase durch ihre Reaktion mit den fluormethylierten Polycyanbenzolen (I) oft erst möglich, zumindest aber stark erleichtert wird, eignen sich die Verbindungen (I) - selbstverständlich erst nach Eichung mit den fraglichen Salzen -hervorragend für eine qualitative Erfassung von Anionen.

Da die aus den fluormethylierten Polycyanbenzolen (I) und Salzen erhältlichen Anlagerungsverbindungen entweder stark gefärbt sind und/oder im UV-Bereich deutlich absorbieren, lassen sich die Verbindungen (I) - nach entsprechender Eichung - auch zur quantitativen Anionenbestimmung, z.B. kolorimetrisch verwenden.

Die Erfindung betrifft daher auch ein Verfahren zur qualitativen und/oder quantitativen Bestimmung von Anionen, wonach man die Salze, die die zu bestimmenden Anionen enthalten, oder die Lösungen dieser Salze mit Lösungen der fluormethylierten Polycyanobenzole der Formel I in Berührung bringt.

Bei dem In-Berührung-bringen von Salzen und fluormethylierten Polycyanobenzolen der Formel I bilden sich stark gefärbte, gegebenenfalls fluoreszierende oder im UV-Licht absorbierende salzartige Addukte, deren Licht- bzw. UV-Absorption im wesentlichen durch die Anionenkomplexe bestimmt wird, die durch die Anlagerung der Anionen der Salze an die fluormethylierten Polycyanobenzole der Formel I entstehen.

Als Vertreter der erfindungsgemäß zu verwendenden fluormethylierten Polycyanobenzole der Formel I seien beispielsweise genannt:
Pentacyano-trifluoromethyl-benzol, 1,2,4,5-Tetracyano-3,6-bis-(trifluoromethyl)-benzol, Pentacyano-difluoromethyl-benzol und Pentacyano-chlorodifluoromethyl-benzol.

Zum Nachweis und zur Bestimmung der Anionen können die fluormethylierten Polycyanobenzole der Formel I in Form von 0,1 - 5 gew.-%igen Lösungen in organischen Lösungsmitteln wie Acetonitril oder Gemischen von organischen und anorganischen Lösungsmitteln wie Acetonitril und Wasser angewendet werden.

Die erfindungsgemäß als Nachweis-Reagentien zu verwendenden fluormethylierten Polycyanobenzole der Formel I eignen sich vor allem zum Sichtbarmachen von Anionen nach ihrer Trennung durch Papier-, Dünnschicht- oder Ionenchromatographie.

Die 1 gew.-%ige Lösung von Pentacyano-trifluoromethyl-benzol in Acetonitril ergibt mit Salzen anorganischer Säuren folgende Färbungen:
mit Kaliumcyanid eine orange, fluoreszierende Färbung,
mit Kaliumrhodanid eine rote Färbung,
mit Natriumcyanat eine gelbe, fluoreszierende Färbung.

Die 1 gew.-%ige Lösung von Pentacyano-trifluoromethyl-benzol in einem Acetonitril/Wasser-(1:1 Volumenteile)-Gemisch ergibt mit Salzen anorganischer Säuren folgende Färbungen:
mit Natriumfluorid, Natriumchlorid und Kaliumbromid gelbe, fluoreszierende Färbungen,
mit Kaliumiodid eine violette Färbung,
mit Natriumcarbonat, Natriumhydroxid und Natriumsulfat orange, fluoreszierende Färbungen;
mit Natriumsulfat eine gelbe, fluoreszierende Färbung,
mit Natriumsulfit eine tiefrote Färbung,
mit Natriumnitrat und Natriumnitrit gelbe Färbungen, und
mit Dinatriumphosphat und Natriumhypophosphit rote Färbungen.

Mit der 1 gew.-%igen Lösung von Pentacyano-trifluoromethyl-benzol in dem Acetonitril/Wasser-(1:1 Volumenteile)-Gemisch werden mit Salzen organischer Säuren, z.B. Salzen von Carbonsäuren, wie Natriumformiat, Natriumacetat, Natriumtartrat, Natriumoxalat gelbe, fluoreszierende Färbungen erhalten.

Im allgemeinen ergeben die Ammoniumsalze mit den fluormethylierten Polycyanobenzolen der Formel I zwar schwächer gefärbte Addukte als die entsprechenden Natriumsalze; da diese Addukte der Ammoniumsalze an die Verbindungen der Formel I jedoch stark im UV-Bereich absorbieren, können auch die Ammoniumsalze mit den erfindungsgemäß zu verwendenden fluormethylierten Polycyanobenzolen der Formel I gut sichtbar gemacht werden.

Das Erkennen und das Bestimmen der Anionen nach der chromatographischen Trennung erfolgt aufgrund der Färbung im sichtbaren Licht oder aufgrund der Fluoreszens unter UV-Licht. Bei der Papier- und Dünnschichtchromatographie können die Chromatogramme mit der Lösung der erfindungsgemäß zu verwendenen fluormethylierten Polycyanobenzole der Formel I besprüht werden. In der Ionenchromatographie können die Lösungen der erfindungsgemäß zu verwendenden Nachweisreagentien nach erfolgter Auftrennung der Ionen hinter der Trennsäule zum Eluat gegeben werden.

Prozentangaben in den nachfolgenden Beispielen beziehen sich auf das Gewicht.

Beispiel 1

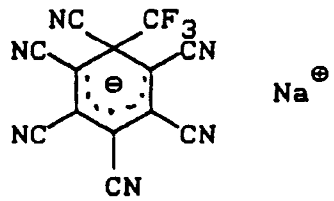

Eine Lösung von 24,3 g (0,1 Mol) α,α,α,2,3,5,6-Heptafluoro-p-tolunitril (J. Org. Chem. 33, 1658) in 250 ml trockenem Acetonitril wird unter Rühren und unter Feuchtigkeitsausschluß mit 24,5 g (0,5 Mol) Natriumcyanid versetzt und 10 Tage bei Raumtemperatur gerührt. Anschließend wird filtriert, mit Acetonitril nachgewaschen und das Filtrat im Rotationsverdampfer bis zu einer Badtemperatur von etwa 30°C eingeengt. Zur Entfernung etwaiger Reste unumgesetzter Heptafluorverbindung wird das eingeengte Filtrat mit 250 ml trockenem Dichlormethan bei Raumtemperatur verrührt, abfiltriert, mit Dichlormethan nachgewaschen und getrocknet. Man erhält 31,0 g (96,8 % der Theorie) orangerotes Natriumsalz der oben angegebenen Konstitution.

Elementaranalyse: $C_{13}F_3N_6Na$

ber.: C 48,77 %, F 17,80 %, N 26,25 %, Na 7,18 %,

gef.: C 48,5 %, F 17,2 %, N 26,4 %, Na 7,0 %,

IR (KBr) in $cm^{-1}$: 2224, 2205, 1563, 1472, 1412, 1359, 1241, 1206, 1175, 1148, 937, 729.

UV/vis (DMF):

$\gamma_{max}$ = 19.400 $cm^{-1}$

$\lambda_{max}$ = 515,5 nm

$\varepsilon_{max}$ = 12.200

75,39 MHz- $^{19}F$-NMR ($d_6$-Aceton):

$\delta_{CF_3}$ = 2,76 ppm, bezogen auf $CF_3$-COOH (extern)

125,77 MHz- $^{13}C$-NMR ($d_6$-Aceton):

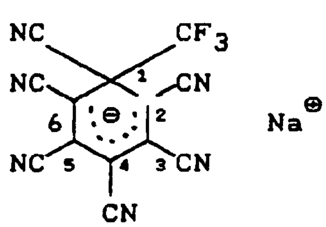

| δ (ppm) | |
|---|---|
| 126.089 | C-3, C-5 |
| 124.323 | $CF_3$; $J_{CF}$ = 290.9 Hz (q) |
| 116.779 | |
| 116.079 | |
| 113.910 | CN |
| 112.745 | |
| 83.309 | C-4 |
| 78.812 | C-2, C-6 |
| 50.623 | C-1; $^3J_{CF}$ = 33.67 Hz (q) |

Fluoreszenz-Spektrum (DMF):

| | |
|---|---|
| max. Emission bei: | 16.900 $cm^{-1}$ |
| max. Excitation bei: | 19.400 $cm^{-1}$ |
| Δ Stokes: | 2.500 $cm^{-1}$ |
| Quantenausbeute: | 0,62 |

Beispiel 2

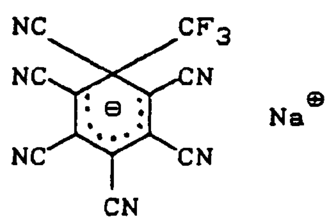

Man verfährt analog Beispiel 1 mit dem Unterschied, daß man die Umsetzung statt bei Raumtemperatur unter Rückfluß durchführt. Nach etwa 20 Stunden ist die Reaktion praktisch beendet. Man erhält das Natriumsalz in einer Ausbeute von ebenfalls über 95 % der Theorie.

Beispiel 3

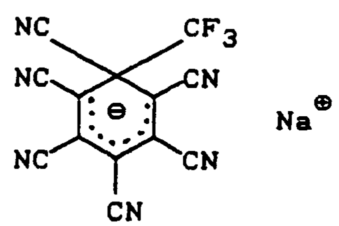

Eine Lösung von 28,5 g (117 mMol) α,α,α,2,3,5,6-Heptafluoro-p-tolunitril in 285 ml trockenem Acetonitril wird unter Rühren und unter Feuchtigkeitsausschluß mit 23,0 g (469 mMol) Natriumcyanid versetzt und 8 Tage bei Raumtemperatur gerührt. Die Aufarbeitung erfolgt wie in Beispiel 1 beschrieben. Man erhält 28,2 g (94 % der Theorie, bezogen auf eingesetztes Natriumcyanid) Natriumsalz der oben angegebenen Konstitution.

Beispiel 4

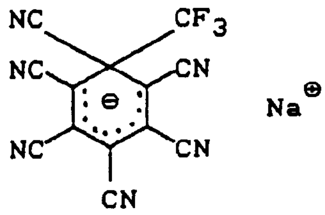

Eine Lösung von 23,6 g (0,1 Mol) Pentafluoro-trifluoromethyl-benzol in 250 ml trockenem Acetonitril wird unter Rühren und unter Feuchtigkeitsausschluß mit 29,4 g (0,6 Mol) Natriumcyanid versetzt und 12 Tage bei Raumtemperatur gerührt. Die Aufarbeitung erfolgt wie in Beispiel 1 beschrieben.

Man erhält 20,8 g (65,0 % der Theorie) Natriumsalz der oben angegebenen Konstitution.

Beispiel 5

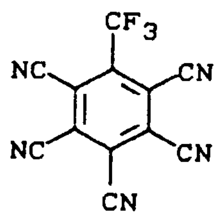

1,00 g (3,125 mMol) des nach Beispiel 1 erhaltenen Natriumsalzes $C_{13}F_3N_6Na$ wird in 5 g Essigsäure gelöst. Nach 12-stündigem Stehen bei Raumtemperatur wird die Lösung mit 50 ml Wasser versetzt, der entstandene Niederschlag abfiltriert, mit Wasser nachgewaschen und getrocknet. Ausbeute: 0,78 g (92,1 % der Theorie) Pentacyano-trifluoromethyl-benzol. Die Verbindung schmilzt noch nicht bei 270°C; sie kann bei 200-220°C/ 0,1 mbar ohne Zersetzung sublimiert werden.

IR (KBr) in $cm^{-1}$: 2250, 1561, 1419, 1327, 1290, 1263, 1204, 1181, 997, 853, 839, 792, 731, 697.

75,39 MHz - $^{19}F$ - NMR ($d_6$ - DMSO):

$\delta CF_3$ = - 19,95 ppm, bezogen auf $CF_3$-COOH (extern)

125,77 MHz- $^{13}C$-NMR ($d_6$-DMSO):

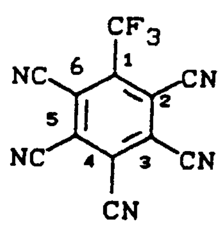

| δ (ppm) | |
|---|---|
| 136.583 | C-1; $^{3}J_{CF}$ = 33,93 Hz (q) |
| 125.292 | C-3, C-5 |
| 123.449 | C-4 |
| 119.527 | $CF_3$; $J_{CF}$ = 277.74 Hz (q) |
| 118.968 | C-2, C-6; $^{4}J_{CF}$ = 1.81 Hz (q) |
| 111.992 | 2 CN an C-2 / C-6 oder C-3 / C-5 |
| 111.595 | CN an C-4 |
| 111.187 | 2 CN an C-3 / C-5 oder C-2 / C-6 |

Beispiel 6

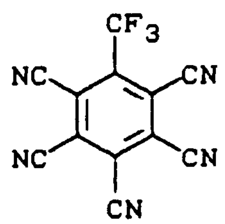

60,5 g (189 mMol) des nach Beispiel 1 hergestellten Natriumsalzes $C_{13}F_3N_6Na$ werden mit ca. 1,5 Liter 1n-wäßriger Salzsäure 24 Stunden bei Raumtemperatur gerührt. Anschließend wird der Niederschlag abfiltriert, mit Wasser gewaschen und getrocknet.

Ausbeute: 48,1 g (93,8 % der Theorie) Pentacyano-trifluoromethyl-benzol, identisch mit der nach Beispiel 5 erhaltenen Verbindung.

Beispiel 7

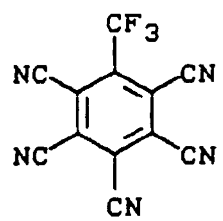

1,00 g (3,125 mMol) des nach Beispiel 1 erhaltenen Natriumsalzes $C_{13}F_3N_6Na$ wird in einer Sublimationsapparatur bei 220°C/0,1 mbar 24 Stunden erhitzt.

Man erhält 0,57 g (67,3 % der Theorie) Pentacyano-trifluoromethyl-benzol, identisch mit der nach Beispiel 5 gewonnenen Verbindung.

Beispiel 8

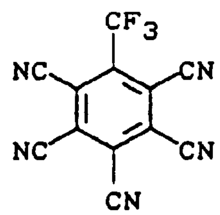

Eine Lösung von 2,43 g (10 mMol) α,α,α,2,3,5,6-Heptafluoro-p-tolunitril in 25 ml trockenem Dimethylformamid wird unter Rühren und unter Feuchtigkeitsausschluß mit 2,53 g (51,6 mMol) Natriumcyanid versetzt, wobei sich die Reaktionsmischung unter sofortiger Rotfärbung erwärmt. Man rührt über Nacht nach und gießt dann in eine Mischung aus 200 ml Wasser und 50 ml konz. Salzsäure. Nach 24-stündigem Rühren bei Raumtemperatur wird der Niederschlag abfiltriert, mit Wasser gewaschen und getrocknet.

Ausbeute: 2,19 g (80,1 % der Theorie) Pentacyano-trifluoromethyl-benzol, identisch mit dem nach Beispiel 5 erhaltenen Produkt.

Beispiel 9

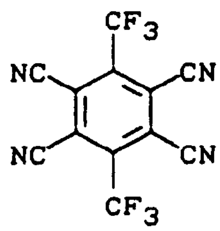

Die Lösung von 5,72 g (20 mMol) 1,2,4,5-Tetrafluoro-3,6-bis(trifluoromethyl)-benzol in 100 ml trockenem Dimethylformamid wird unter Rühren und unter Feuchtigkeitsausschluß mit 4,90 g (100 mMol) Natriumcyanid versetzt, wobei sich die Reaktionsmischung unter sofortiger Rotfärbung erwärmt. Man kühlt anfangs mit Eiswasser, dann rührt man über Nacht bei Raumtemperatur nach. Anschließend wird das Reaktionsgemisch unter Rühren in eine Mischung aus 500 ml Wasser und 500 ml konzentrierter Salzsäure eingegossen. Nach einstündigem Nachrühren wird der Niederschlag abfiltriert und mit Wasser gewaschen, bis das Filtrat farblos abläuft. Nach dem Trocknen erhält man 4,95 g (78,8 % der Theorie) 1,2,4,5-Tetracyano-3,6-bis(trifluoromethyl)-benzol. Die Verbindung schmilzt noch nicht bei 270°C; sie kann bei 200°C/0,1 mbar ohne Zersetzung sublimiert werden.

IR (KBr) in $cm^{-1}$: 2253, 1444, 1422, 1314, 1206, 1180, 1136, 856, 678.

Beispiel 10

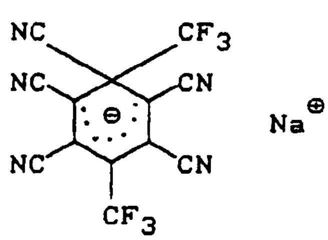

Eine Lösung von 5,72 g (20 mMol) 1,2,4,5-Tetrafluoro-3,6-bis(trifluoromethyl)-benzol in 80 ml trockenem Acetonitril wird unter Rühren und unter Feuchtigkeitsausschluß mit 3,92 g (80 mMol) Natriumcyanid versetzt und 10 Tage bei Raumtemperatur gerührt. Anschließend wird das Reaktionsgemisch wie in Beispiel 1 beschrieben aufgearbeitet.

Man erhält 3,40 g (58,6 % der Theorie, bezogen auf eingesetztes Natriumcyanid) rotes Natriumsalz $C_{13}F_6N_5Na$ der oben angegebenen Konstitution.

IR (KBr) in $cm^{-1}$: 2206, 1544, 1431, 1378, 1292, 1239, 1207, 1146, 1009, 892, 730.

UV/vis (DMF):

$\gamma_{max}$ = 20.100 $cm^{-1}$

$\lambda_{max}$ = 497,5 nm

$\varepsilon_{max}$ = 8.300

Beispiel 11

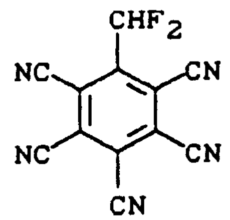

Eine auf etwa -40°C gekühlte Lösung von 10,9 g (0,05 Mol) Difluoromethyl-pentafluoro-benzol in 200 ml trockenem Dimethylformamid wird unter Rühren und unter Feuchtigkeitsausschluß mit 12,25 g (0,25 Mol) Natriumcyanid versetzt. Schon nach 5-10 Minuten und einer Innentemperatur von -40°C ist das Reaktionsgemisch kräftig orangerot gefärbt. Man rührt weitere 2 Stunden bei Temperaturen unter 0°C weiter; dann läßt man das Reaktionsgemisch über Nacht allmählich auf Raumtemperatur erwärmen und rührt es noch weitere 10 Tage bei Raumtemperatur. Anschließend gießt man das Reaktionsgemisch in etwa 2 Liter 1n Salzsäure. Die Mischung wird über Nacht bei Raumtemperatur gerührt. Dann wird der Niederschlag abfiltriert, mit Wasser gewaschen und getrocknet.

Man erhält in guter Ausbeute Difluoromethyl-pentacyano-benzol, das bei 270°C noch nicht schmilzt und bei 200°C/0,1 mbar ohne Zersetzung sublimiert werden kann.

$^1$H-NMR ($d_6$-DMSO): $\delta$ = 7,6 ppm (t, J = 50,0 Hz)

IR (KBr) in $cm^{-1}$: 2248, 1565, 1431, 1378, 1343, 1307, 1277, 1136, 1077, 1043, 896, 834, 790, 704, 627.

Beispiel 12

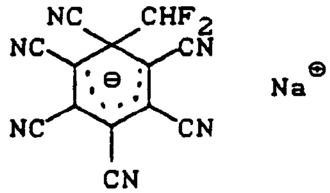

Man verfährt wie in Beispiel 4 beschrieben mit dem Unterschied, daß anstelle von 0,1 Mol Pentafluoro-trifluoromethyl-benzol 21,8 g (0,1 Mol) Difluoromethyl-pentafluoro-benzol eingesetzt werden. Man erhält in guter Ausbeute das organgerote Natriumsalz $C_{13}HF_2N_6Na$ der oben angegebenen Konstitution.

IR (KBr) in $cm^{-1}$: 2198, 1563, 1476, 1406, 1356, 1239, 1206, 1128, 1088, 748, 728.

UV/vis (DMF):

$\gamma_{max}$ = 19.350 $cm^{-1}$

$\lambda_{max}$ = 516,8 nm

$\varepsilon_{max}$ = 9.400

Beispiel 13

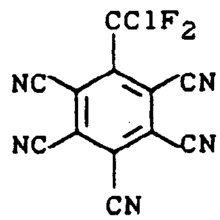

Man verfährt wie in Beispiel 11 beschrieben mit dem Unterschied, daß anstelle von 0,05 Mol Difluoromethyl-pentafluoro-benzol 12,63 g (0,05 Mol) Chlorodifluoromethyl-pentafluoro-benzol eingesetzt werden.

Man erhält Chlorodifluoromethyl-pentacyano-benzol, das bei 270°C noch nicht schmilzt und bei 200°C/0,1 mbar ohne Zersetzung sublimiert werden kann.

IR (KBr) in $cm^{-1}$: 2247, 1415, 1310, 1283, 1161, 1018, 921, 846, 792, 753, 669, 623.

Anwendungsbeispiele

Anwendung 1

Auf einer Cellulose-Platte (z.B. Merck, Art-Nr. 5786) werden 2 µl von Lösungen mit je 10, 20, 50, 100, 200, 500 mg/100 ml-Lösung Malonsäure, Bernsteinsäure, Glutarsäure, Adipinsäure, Korksäure und Sebacinsäure in i-Propanol : Wasser = 4:1 Volumenteile aufgetragen.

Die chromatographische Trennung erfolgt durch Entwickeln mit n-Propanol : Methanol : 25 %igem wäßrigem Ammoniak = 6:1:3 Volumenteile.

Nach dem Besprühen mit einer 1 %igen Lösung von Pentacyano-trifluoromethyl-benzol (aus Beispiel 5) in Acetonitril werden die als Ammoniumsalze vorliegenden Säuren unter UV-Licht (366 nm) erkennbar. Nachweisgrenze: 1 µg. Auf diese Weise wurden für die einzelnen Säuren folgende $R_f$-Werte ermittelt: 0,16 für Malonsäure; 0,27 für Bernsteinsäure; 0,32 für Glutarsäure; 0,39 für Adipinsäure; 0,45 für Korksäure und 0,53 für Sebacinsäure.

Im Gegensatz zur Anfärbung der Anionen mit Methylrot, die bereits nach wenigen Minuten verschwindet, bleibt die Sichtbarkeit der Anionen unter UV nach Anfärben mit Pentacyano-trifluoromethyl-benzol tagelang erhalten.

Anwendung 2

Auf einer Cellulose-Platte (z.B. Merck, Art-Nr. 5786) werden Lösungen von Ameisensäure, Essigsäure und Propionsäure in Aceton aufgetragen. Die Auftragungen enthalten je 2 µg, 4 µg, 10 µg, 20 µg und 40 µg jeder Säure.

Die chromatographische Trennung erfolgt durch Entwickeln mit n-Propanol : Methanol : 25 %igem wäßrigem Ammoniak = 6:1:3 Volumenteile.

Nach dem Besprühen mit einer 1 %igen Lösung von Pentacyano-trifluoromethyl-benzol in Acetonitril werden die als Ammoniumsalze vorliegenden Säuren unter UV-Licht (366 nm) erkennbar.

Die Nachweisgrenzen für die einzelnen Säuren betragen: 20 µg für Ameisensäure, 4 µg für Essigsäure und 2 µg für Propionsäure. Folgende $R_f$-Werte wurden für die einzelnen Säuren ermittelt: 0,47 für Ameisensäure, 0,52 für Essigsäure unf 0,56 für Propionsäure.

Anwendung 3

Auf Papier, z.B. Schleicher & Schüll, Nr. 2040 b werden je 2 µl einer 1 %igen Lösung von Natriumchlorid, Natriumbromid, Natriumiodid und Kaliumrhodanid aufgetragen.

Die chromatographische Entwicklung erfolgt mit Butanol : Pyridin : 25 %igem wäßrigem Ammoniak = 2:1:2 Volumenteile.

Nach dem Besprühen mit einer 1 %igen Lösung von Pentacyano-trifluoromethyl-benzol in Acetonitril erscheint jeweils ein gelber Fleck bei $R_f$-Wert = 0,2, der auf NaOH oder KOH zurückgeht. Im UV-Licht (366 nm)

werden weitere Flecke erkennbar, und zwar bei $R_f$ = 0,32 für Chlorid, 0,43 für Bromid, 0,54 für Iodid und 0,62 für Rhodanid.

Wird die Trennung auf einer Celluloseplatte durchgeführt, so betragen die $R_f$-Werte 0,46 für Chlorid, 0,55 für Bromid, 0,66 für Iodid und Rhodanid. Auf der Celluloseplatte können auch Sulfat ($R_f$-Wert 0,32) und Sulfid ($R_f$-Wert 0,36) sichtbar gemacht werden.

Anwendung 4

Auf eine Kieselgel-Platte, z.B. Kieselgel 60 ohne Fluoreszensindikator (Merck Art-Nr. 5721), werden Lösungen von Chloressigsäure, Glykolsäure, Ameisensäure und Malonsäure aufgetragen. Die Auftragungen enthalten 1 µg, 5 µg, 10 µg und 50 µg jeder Säure.

Die chromatographische Entwicklung erfolgt in der Sandwichkammer mit Methanol : Ethanol : i-Propanol : i-Butanol : 10 %igem wäßrigem Ammoniak = 10:15:20:30:25 Volumenteile.

Nach dem Besprühen mit einer 1 %igen Lösung von Pentacyano-trifluoromethyl-benzol in Acetonitril sind die als Ammoniumsalze vorliegenden Säuren unter UV-Licht (366 nm) bis 1 µg herunter sichtbar. Die $R_F$-Werte betragen: 0,16 für Malonsäure, 0,36 für Chloressigsäure, 0,44 für Glykolsäure und 0,59 für Aminoessigsäure.

Anwendung 5

Auf einer Celluloseplatte, z.B. Merck, Art-Nr. 5786, werden je 2 und 10 µl von 0,1 %igen wässrigen Lösungen von Natriumsulfit, Natriumsulfat, Natriumthiosulfat und Natriumsulfid aufgetragen.

Die chromatographische Trennung erfolgt durch Entwickeln mit Butanol : Pyridin : 25 %igem wäßrigem Ammoniak = 2:1:2 Volumenteile.

Nach dem Besprühen mit einer 1 %igen Lösung von Pentacyano-trifluoromethyl-benzol in Acetonitril erscheinen Natriumsulfit und Natriumthiosulfat als rote bzw. braune Flecke (Nachweisgrenze 2 µg), Natriumsulfid als gelber Fleck (Nachweisgrenze 10 µg), Natriumsulfat ist unter UV-Licht sichtbar (Nachweisgrenze 10 µg).

**Patentansprüche**

1. Fluormethylierte Polycyanbenzole der Formel

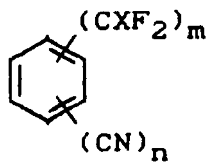

(I),

in der

X für Wasserstoff, Fluor oder Chlor steht,
m 1 oder 2 ist und
n (6 -m) ist.

2. Verfahren zur Herstellung der fluormethylierten Polycyanbenzole nach Anspruch 1, dadurch gekennzeichnet, daß man fluormethylierte Fluorbenzole der Formel

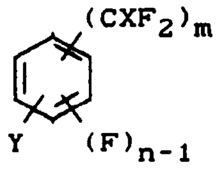

(II),

in der X, m und n die in Anspruch 1 angegebene Bedeutung haben und
Y für Fluor oder CN steht,

mit Alkalicyaniden in organischen Verdünnungsmitteln zu salzartigen Addukten der Formel

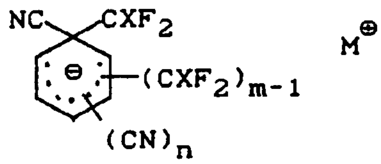 (III)

umsetzt, in der X, m und n die oben angegebene Bedeutung haben und $M^{\oplus}$ für ein Alkalimetall-Kation steht,

und dieses Addukt, gegebenenfalls ohne es zu isolieren, durch Behandeln mit Säuren oder durch Erhitzen auf Temperaturen von 150-300°C, vorzugsweise im Vakuum, in die Verbindungen der Formel (I) überführt.

**3.** Salzartige Addukte der Formel

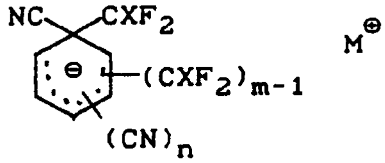 (III),

in der

| | |
|---|---|
| X | für Wasserstoff, Fluor oder Chlor steht, |
| m | 1 oder 2 ist, |
| n | (6 -m) ist und |
| $M^{\oplus}$ | für ein Alkalimetall-Kation steht. |

**4.** Verwendung der fluormethylierten Polycyanobenzole nach Anspruch 1 zum Sichtbarmachen und/oder zur qualitativen bzw. quantitativen Bestimmung von Anionen.

## Claims

**1.** Fluoromethylated polycyanobenzenes of the formula

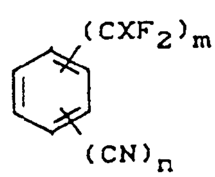 (I),

in which

| | |
|---|---|
| X | represents hydrogen, fluorine or chlorine, |
| m | is 1 or 2 and |
| n | is (6 - m). |

**2.** Process for preparing the fluoromethylated polycyanobenzenes according to Claim 1, characterized in that fluoromethylated fluorobenzenes of the formula

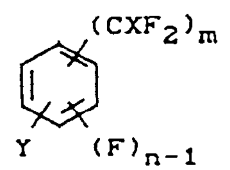

(II),

in which X, m and n have the meanings specified in Claim 1 and

Y represents fluorine or CN,

are reacted with alkali metal cyanides in organic diluents to form salt-like adducts of the formula

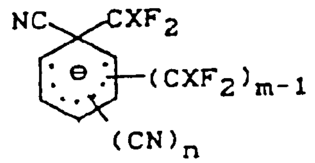

(III),

in which X, m and n have the specified meanings and $M^{\oplus}$ represents an alkali metal cation, and this adduct, optionally without being isolated, is converted by treatment with acids or by heating to temperatures of 150-300°C, preferably in vacuo, into the compounds of the formula (I).

3. Salt-like adducts of the formula

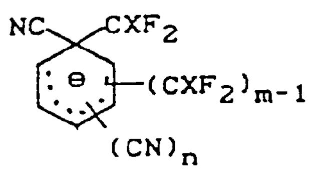

(III),

in which

X represents hydrogen, fluorine or chlorine,

m is 1 or 2,

n is (6 - m) and

$M^{\oplus}$ represents an alkali metal cation.

4. Use of the fluoromethylated polycyanobenzenes according to Claim 1 for the visualization and/or for the qualitative and/or quantitative determination of anions.

## Revendications

1. Polycyanaobenzènes fluorométhylés de formule :

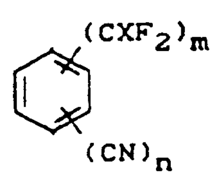

(I),

dans laquelle

X représente l'hydrogène, le fluor ou le chlore,

m est égal à 0 ou 1, et

n est égal à (6 - m).

2. Procédé de préparation des polycyanobenzènes fluorométhylés selon la revendication 1, caractérisé en ce que l'on fait réagir des fluorobenzènes fluorométhylés de formule :

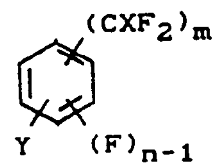

(II),

dans laquelle
X, m et n ont les significations indiquées dans la revendication 1, et
Y représente le fluor ou CN,
avec des cyanures alcalins dans des diluants organiques, ce qui donne des adducts salins de formule :

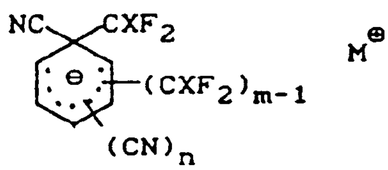

(III)

dans laquelle
X, m et n ont les significations indiquées ci-dessus et $M^{\oplus}$ représente un cation de métal alcalin,
et on concertit ces adducts, éventuellement sans les isoler, en les composés de formule I par traitement à l'aide d'acides ou par chauffage à des températures de 150 à 300°C, de préférence sous vide.

3. Adducts salins de formule :

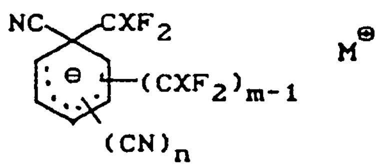

(III),

dans laquelle
X représente l'hydrogène, le fluor ou le chlore,
m est égal à 1 ou 2,
n est égal à (6 - m), et
$M^{\oplus}$ représente un cation de métal alcalin.

4. Utilisation des polycyanobenzènes fluorométhylés selon la revendication 1, pour la détection et/ou l'analyse qualitative ou quantitative d'anions.